**Europäisches Patentamt**

(19) **European Patent Office**

**Office européen des brevets**

(11) Numéro de publication : **0 105 290**
**B1**

(12) # FASCICULE DE BREVET EUROPÉEN.

(45) Date de publication du fascicule du brevet :
**22.10.86**

(51) Int. Cl.⁴ : **A 01 N 1/02**, A 61 L 27/00, A 61 F 2/24

(21) Numéro de dépôt : **83900937.0**

(22) Date de dépôt : **22.03.83**

(86) Numéro de dépôt international :
**PCT/FR 83/00057**

(87) Numéro de publication internationale :
**WO/8303335 (13.10.83 Gazette 83/24)**

(54) **TISSU BIOLOGIQUE GREFFABLE ET PROCEDE POUR SA PREPARATION.**

(30) Priorité : **23.03.82 FR 8204892**

(43) Date de publication de la demande :
**18.04.84 Bulletin 84/16**

(45) Mention de la délivrance du brevet :
**22.10.86 Bulletin 86/43**

(84) Etats contractants désignés :
**CH DE GB LI**

(56) Documents cités :
**EP-A- 0 065 827**
**DE-A- 2 207 787**
**FR-A- 1 393 519**
**FR-A- 2 298 312**
**Chemical Abstracts, volume 95, no. 17, 26 octobre 1981 (Columbus Ohio, US) H. Karaki et al. "Rabbit aortic contractile responses and calcium-45 retention in tris and bicarbonate buffers", voir page 443, résumé no. 148098v, Arch. Int. Pharmacodyn. Ther. 1981, 252(1), 29-39**
**Chemical Abstracts, vol. 93, no. 13, 29 septembre 1980 (Columbus Ohio, US) B. Altura et al. "Adverse effects of artificial buffers on contractile responses of arterial and venous smooth muscle",voir page 125, résumé no. 126343c, Br. J. Pharmacol. 1980, 69(2), 207-14**
**Chemical Abstracts, vol. 93, no. 22, 22 décembre 1980 (Columbus, Ohio, US) Altura, Burton et al.: "Adverse effects of Tris, HEPES and MOPS buffers on contrac-**

(73) Titulaire : **AMERICAN HOSPITAL SUPPLY CORPORA-TION**
**One American Plaza**
**Evanston, IL 60201 (US)**

(72) Inventeur : **Carpentier, Alain F.**
**96 rue Didot**
**F-75014 Paris (FR)**
Inventeur : **Carpentier, Sophie**
**96 rue Didot**
**F-75014 Paris (FR)**
Inventeur : **Nashef, Aws S.**
**2824 Corvo Place**
**Costa Mesa CA 92626 (US)**

(74) Mandataire : **Hirsch, Marc-Roger**

tile responses of arterial and venous smooth muscle induced by prostaglandins", voir page 155, résumé no. 231695s, Prostaglandins Med. 1980, 5(2), 123-30

Chemical Abstracts, vol. 85, no. 13, 27 septembre 1976 (Columbus, Ohio, US) H. Luoma et al.: "The effect of magnesium and fluoride on nephrocalcinosis and aortic calcification in rats given high sucrose diets with added phosphates", voir page 490, résumé no. 92509d, Calcif. Tissue Res. 1976, 20(3), 291-302

Chemical Abstracts, vol. 74, no. 11, 15 mars 1971 (Columbus, Ohio, US) W.M. Britton et al.: "Aorta and other soft tissue calcification in the magnesium-deficient rat", voir page 130, réf. 51080m, J. Nutr. 1970 100(12), 1501-6

## Description

La présente invention concerne un procédé pour le traitement et la conservation d'un tissu biologique destiné à être greffé. Elle se rapporte également à une bioprothèse stable obtenue à partir d'un tel tissu. En fait, elle se rapporte à un procédé permettant d'empêcher la calcification des tissus pendant leur conservation et d'obtenir à partir de.tels tissus des bioprothèses stables ayant une tendance réduite à la calcification.

La conservation par le glutaraldéhyde des tissus biologiques, en particulier des valvules cardiaques bioprothétiques porcines, a permis de :

a) éviter les mauvaises performances des tissus valvulaires greffés conservés par le formaldéhyde ;

b) cesser de réaliser des homogreffes valvulaires ; et

c) éviter l'emploi indésirable des anticoagulants nécessaires pour empêcher une thromboembolie associée à l'emploi des valvules cardiaques non bioprothétiques (mécaniques) en particulier chez l'enfant.

Cependant, comme c'est le cas d'autres découvertes importantes semblables, il semble que les bioprothèses conservées par le glutaraldéhyde posent des problèmes qui leur sont propres.

Bien que les valvules de Carpentier et autres conservées par le glutaraldéhyde, qui sont relativement inertes du point de vue biologique, présentent une excellente longévité dans la plupart des cas, leur emploi continu présente des inconvénients graves, tels qu'une fatigue tissulaire et une tendance à la calcification. De plus, on considérait au départ que les enfants et les adolescents seraient les sujets tirant le plus grand profit des valvules cardiaques bioprothétiques conservées par le glutaraldéhyde, grâce à la suppression des anticoagulants que nécessitent les prothèses mécaniques. Les résultats d'un nombre croissant d'études cliniques récentes montrent qu'une calcification grave de ces tissus s'accompagnant d'une défaillance relativement rapide, s'observe chez les enfants et les adolescents. Donc, malgré leur longévité prolongée et la fréquence globale réduite des complications, ces valvules conservées par le glutaraldéhyde sont considérées par certains comme inappropriées à l'emploi chez l'enfant.

La calcification tissulaire demeure en grande partie mystérieuse ; cependant on a précédemment montré que divers facteurs, tels que les anomalies du métabolisme du calcium, l'âge, l'alimentation, la dégénérescence de composants tissulaires tels que le collagène et les turbulences, interviennent tous dans une certaine mesure. Récemment, l'existence d'un amino-acide spécifique fixant le calcium, qui s'accumule après l'implantation des xénogreffes porcines conservées par le glutaraldéhyde, a été établie et on a pensé qu'il jouait un rôle dans la calcification. Bien que la calcification s'accompagne de dégradations des fibres de collagène du tissu greffé traité par le glutaraldéhyde, on ne sait pas si la calcification dystrophique est la cause ou le résultat de la dégénérescence tissulaire. Néanmoins, on a poursuivi les efforts pour élucider la source du problème de la calcification des tissus greffés en espérant y porter rapidement remède. A ce jour, ni la source ni la cause de la calcification des greffons biologiques, ni des mesures appropriées pour éviter ou réduire la calcification des greffons biologiques n'ont été établies.

Selon l'invention, on a déterminé une cause sous-jacente de la calcification des greffons biologiques et, en particulier, des bioprothèses valvulaires conservées par le glutaraldéhyde. De plus, on a simultanément mis au point des procédés permettant de réduire ou d'atténuer de façon efficace la calcification des tissus biologiques greffés.

Une des causes sous-jacentes de la calcification des bioprothèses valvulaires, notée pour la première fois dans.les études immédiates des demandeurs, est la présence de phosphate en contact avec les tissus avant la greffe en des quantités qui entretiennent la calcification après la greffe. En fait aucune des méthodes décrites dans l'art antérieur même celles faisant état de l'effet inhibiteur de $Mg^{2+}$ sur la calcification du tissu animal (Chem. Abstr. 74, 52080m et Chem. Abstr. 85, 92509d) ne conduisent à ce résultat inattendu et totalement surprenant de donner lieu à une diminution ou atténuation prolongée de la calcification après la greffe.

Les teneurs en phosphate normalement observées dans les milieux d'expédition, tels que les solutions équilibrées (solution de Hanks, etc.), le plasma, et le tampon salé phosphaté 0,01 à 0,10 M (PBS), utilisés classiquement dans les solutions de fixation à base de glutaraldéhyde, entretiennent tous à des degrés divers la calcification des tissus. A ce jour, les effets nuisibles du phosphate en contact avec les greffons biologiques n'ont pas été appréciés et, par conséquent, les chercheurs, les cliniciens et les fabricants ignorent les conséquences indésirables que provoque le traitement de ces greffons par des solutions phosphatées ; en particulier du fait que des solutions phosphatées, telles que la solution de Hanks, le PBS et le glutaraldéhyde-PBS, ont été couramment utilisées et sont très recommandées. On comprend donc pourquoi on ait parfois recommandé, au lieu du tampon PBS, d'utiliser pour le stockage des tissus des tampons bicarbonate (ayant des capacités tampons et des gammes de pH semblables), l'emploi dans ce cas d'un milieu ne contenant pas de phosphate ne résultant pas d'une intention délibérée. De plus, dans certains cas, même les milieux de conservation des tissus tamponnés au bicarbonate ont des teneurs élevées en phosphate. Comme les conséquences fâcheuses du maintien des greffons tissulaires en contact avec un phosphate n'étaient pas connues, les cliniciens ou les fabricants des bioprothèses ne cherchaient pas délibérément à éviter le contact des tissus avec les solutions de phosphate.

L'invention concerne des procédés pour traiter un tissu biologique avant la greffe afin de réduire de façon efficace la calcification des tissus biologiques greffés. Ces procédés réduisent de façon avantageuse la tendance à la calcification des bioprothèses et résolvent certains des problèmes associés à la longévité des xénogreffes de valvules cardiaques.

L'invention concerne un procédé amélioré pour conserver un tissu biologique avant la greffe qui atténue ou réduit la calcification après la greffe. Selon un mode de réalisation, le procédé consiste à mettre en contact ledit tissu avec une solution isotonique hypophosphatée efficace pour réduire la calcification du tissu, ladite solution isotonique comprenant au moins un sel ou tampon physiologiquement acceptable ayant un pH compris entre 7,0 et 7,6 et plus de 0,003 % en poids d'un cation divalent autre que le calcium entrant en compétition vis-à-vis des sites de fixation du calcium, ledit tissu étant conservé dans ladite solution jusqu'à son utilisation. Ladite solution a une teneur en phosphate abaissée à une valeur efficace pour réduire la calcification dudit tissu après la greffe, cette solution ne détruisant pas et ne déstabilisant pas le tissu, d'une part et le cation divalent qu'elle contient s'oppose par compétition à la fixation du calcium, ce qui est également efficace pour réduire la calcification du tissu après la greffe.

L'invention va maintenant être décrite de façon détaillée.

L'invention a pour objet de rendre résistants à la calcification divers types de tissus biologiques greffables provenant de nombreuses sources animales et de nombreuses régions de l'organisme. Ainsi, le tissu peut provenir entre autres de bovins, de porcins, de chevaux ou de lapins ; et il peut être constitué de tendons, de ligaments, de valvules cardiaques ou de tissus utilisés pour réaliser des valvules cardiaques, tels que la dure-mère et le péricarde. Egalement, l'invention peut s'appliquer à un tissu utilisé pour réaliser des greffes en plaque telles que des plaques cutanées, des plaques péricardiques, des plaques aortiques et des tympans. Selon l'invention, on n'a pas observé de différence significative de la vitesse de calcification entre le péricarde de porc et les tissus des valvules aortique, tricuspide et mitrale de porc.

Bien que l'on se soit principalement intéressé aux préparations tissulaires qui sont fixées ou tannées, telles que les valvules cardiaques traitées au glutaraldéhyde, les tissus conservés non fixés bénéficient de l'invention. Selon un mode de réalisation préféré de l'invention, des valvules cardiaques ou du tissu péricardique de porc fixés dans le glutaraldéhyde et traités puis implantés sous la peau de rats et de lapins ont bénéficié d'une atténuation ou réduction prolongée inattendue de la calcification après l'implantation. Cette atténuation prolongée de la calcification constitue un procédé d'accroissement de la longévité du tissu greffé, en particulier des valvules cardiaques bioprothétiques.

Selon le procédé de l'invention, un tissu biologique, maintenu avant la greffe dans une solution hypophosphatée, présente de façon avantageuse une diminution ou une atténuation prolongée de la calcification après la greffe. Selon un mode de réalisation de l'invention, les solutions considérées comme hypophosphatées sont celles ayant des teneurs en phosphate inférieures à une valeur entretenant la calcification ; c'est donc une teneur inférieure à celles actuellement utilisées pour lesquelles on n'observe pas de diminution ou d'atténuation notable de la calcification. On considère que les solutions hypophosphatées sont constituées des solutions contenant nettement moins de phosphate que les solutions tampons salées phosphatées (PBS) 0,01 à 0,2 M utilisées classiquement pour la préparation des tissus avant la greffe et ces solutions hypophosphatées se sont révélées efficaces pour atténuer ou réduire la calcification après la greffe. Dans un mode de réalisation préféré de l'invention, ces teneurs réduites en phosphate sont inférieures à la gamme des teneurs en phosphate normalement présentes dans le plasma ou les solutions salées équilibrées telles que les solutions de Hanks et de Earle qui sont comprises entre environ 0,001 et environ 0,002 M. De plus, il s'est révélé particulièrement préférable d'éliminer à fond par rinçage du tissu hôte après le prélèvement pour éliminer ou réduire fortement la quantité de phosphate du sang venant en contact avec le tissu.

Les solutions pratiquement dépourvues de phosphate sont des solutions qui ne contiennent que des traces de phosphate, telles que les quantités correspondant aux impuretés présentes dans la plupart des composés chimiques utilisés pour la préparation des solutions classiques de traitement des tissus. Les solutions de tampon HEPES préparées selon l'invention et décrites ci-après contiennent environ 2 à 4 ppm d'ions phosphates. De plus, certaines solutions de glutaraldéhyde préparées selon l'invention et décrites ci-après contiennent environ 2 à 23 ppm d'ions phosphates utilisés comme stabilisants par certains fabricants. Selon l'invention, ces traces ou résidus de phosphate sont négligeables. Selon l'invention, on préfère particulièrement les solutions pratiquement dépourvues de phosphate.

On évite que le tissu avant la greffe soit placé dans un environnement phosphaté entretenant la calcification, mais de plus on préfère utiliser des solutions qui ne détruisent pas ou ne déstabilisent pas les tissus. On a par exemple constaté que des valvules cardiaques fixées dans du formaldéhyde à 1 % dans l'eau ne se calcifient pas après avoir été greffées à des rats tandis que les mêmes valvules fixées dans du formaldéhyde à 1 % dans du PBS présentent une calcification considérable pendant la même période. Cependant, les valvules traitées par la solution aqueuse de formaldéhyde présentent une dégénérescence appréciable après environ 1 mois. Le traitement des valvules par le formaldéhyde aqueux, malgré l'absence de phosphate, altère la stabilisation du tissu après la greffe et doit être évité.

Les solutions hypophosphatées de l'invention comprennent l'eau distillée, des solutions tampons ou des compositions compatibles avec les tissus, telles que les solutions salées ou leurs combinaisons, telles que les solutions salées tamponnées. Dans un mode de réalisation préféré, la solution hypophosphatée est une solution salée non tamponnée et dans un mode de réalisation particulièrement préféré, c'est une solution salée tamponnée. Dans toutes ces solutions, on préfère opérer dans une gamme d stabilisant le tissu, c'est-à-dire dans une gamme de pH n'ayant pas d'effet nocif vis-à-vis des composants des tissus. Une gamme de pH préférée est comprise entre environ 7,0 et environ 7,6 et mieux entre environ 7,1 et environ 7,4. Le pH que l'on préfère tout particulièrement selon l'invention a une valeur de 7,3.

Les tampons utilisés selon l'invention sont de préférence stables, inertes vis-à-vis du processus de stabilisation et ont un pouvoir tampon suffisant pour maintenir un pH acceptable en particulier pendant la fixation des tissus. Le choix du tampon approprié et de sa concentration dépend des conditions particulières de préparation des tissus auxquelles plusieurs fabricants ont apporté des variantes. Les tampons préférés selon l'invention comprennent les tampons borate, carbonate, bicarbonate, cacodylate (non toxiques chez l'animal) et d'autres tampons synthétiques artificiels ou organiques tels que l'HEPES (acide N-2-hydroxyéthylpipérazine-N'-2-éthanesulfonique), le MOPS (acide morpholinepropanesulfonique) et le PIPES (acide 1,4-pipérazinediéthanesulfonique). Les tissus préparés dans le tampon HEPES présentent de façon avantageuse une diminution importante de la calcification après la greffe et on préfère donc particulièrement ce tampon dans l'invention.

Dans l'invention, on choisit essentiellement la concentration du tampon dans la solution hypophosphatée pour maintenir le tissu dans un environnement hypophosphaté ou pour remplacer de façon efficace le phosphate déjà présent dans les tissus tout en ajustant simultanément le pH de la solution. Il est important que la quantité de ce tampon utilisé seul ou en combinaison avec des solutions telles qu'une solution salée, soit efficace pour amener ou maintenir le tissu immergé dans un environnement hypophosphaté. Dans un mode de réalisation préféré, on utilise un tampon ayant une concentration d'environ 0,001 à environ 0,10 M en HEPES. Dans un mode de réalisation particulièrement préféré, on utilise un tampon ayant une concentration en HEPES d'environ 0,002 à environ 0,050 M. Le tampon que l'on préfère particulièrement pour la fixation par le glutaraldéhyde a une concentration en HEPES d'environ 0,02 M.

De préférence, les solutions tamponnées ou non tamponnées utilisées selon l'invention doivent être inertes vis-à-vis du processus de stabilisation des tissus que provoquent des agents fixateurs tels que le glutaraldéhyde. Ils ne doivent donc pas réagir avec l'agent fixateur, ni empêcher l'agent fixateur de réaliser la fixation appropriée des tissus. On sait que des tampons contenant des amines primaires et secondaires, tels que le tris(hydroxyméthyl)aminométhane (TRIS), réagissent avec les groupes aldéhydes du glutaraldéhyde et gênent ainsi le processus normal de stabilisation des tissus. Des tampons, tels que le TRIS, bien qu'ils soient pratiquement dépourvus de phosphate, nuisent à la stabilisation des tissus après la greffe et doivent donc être évités.

Selon l'invention, une exposition de courte durée du tissu biologique à une solution hypophosphatée ou pratiquement dépourvue de phosphate est inefficace pour réduire la calcification. Selon un mode de réalisation de l'invention, on doit maintenir le tissu biologique dans une solution fortement hypophosphatée entre le moment où l'on met le tissu hors de contact avec un phosphate dans l'un quelconque des divers stades et le moment précédant immédiatement la greffe. Par exemple, on a constaté qu'un tissu porcin traité avec une solution hypophosphatée ou une solution pratiquement dépourvue de phosphate après le prélèvement et pendant l'expédition présente une calcification importante si l'on utilise des solutions entretenant la calcification lors des stades de fixation et des stades suivant la fixation de la préparation. D'autre part, on a constaté que des tissus traités par le PBS, qui est une solution entretenant la calcification, après le prélèvement et pendant l'expédition, présentent une calcification réduite si l'on utilise des solutions pratiquement dépourvues de phosphate dans les stades de fixation et les stades suivant la fixation de la préparation. Un tissu traité avec des solutions pratiquement dépourvues de phosphate après le prélèvement, pendant l'expédition, pendant la fixation et les stades de conservation et de stérilisation après la fixation, présente la réduction maximale de la calcification.

Selon un mode de réalisation de l'invention, on préfère maintenir le tissu biologique dans une solution hypophosphatée pendant la période suivant la fixation, c'est-à-dire pendant la période de conservation et de stérilisation, par exemple dans le formaldéhyde, qui suit la fixation et le moment qui précède immédiatement la greffe. On préfère particulièrement mettre le tissu biologique en contact avec une solution fortement hypophosphatée pendant la fixation et maintenir le tissu en contact avec une telle solution hypophosphatée jusqu'au moment qui précède immédiatement la greffe. De préférence, la solution hypophosphatée est un composant de la solution de fixation. On préfère tout particulièrement mettre le tissu en contact immédiatement après le prélèvement avec une solution hypophosphatée et maintenir le tissu dans une solution hypophosphatée pendant l'expédition, la fixation et le stockage et la stérilisation qui suivent la fixation jusqu'au moment qui précède immédiatement la greffe.

Dans un mode de réalisation préféré, le tissu est mis en contact avec une solution fortement hypophosphatée pendant la fixation, cette solution étant un composant de la solution de fixation. On préfère, selon l'invention, des solutions salées contenant du glutaraldéhyde, tamponnées ou non tamponnées, contenant de préférence environ 0,2 à 6,0 % en poids de glutaraldéhyde et mieux environ 5 à environ 0,7 % en poids de glutaraldéhyde. Une solution de fixation que l'on préfère particulièrement est constituée de solution salée contenant environ 0,5 à 0,7 % en poids de glutaraldéhyde. Une solution de fixation pratiquement dépourvue de phosphate constituée de solution salée tamponnée 0,02 M en HEPES et contenant environ 0,625 % en poids de glutaraldéhyde à un pH d'environ 7,1 à environ 7,5, s'est révélée efficace pour réduire la calcification après la greffe et constitue donc un mode de réalisation particulièrement préféré de l'invention.

On envisage de plus que le tissu biologique expédié et/ou fixé en présence de quantités de phosphate entretenant la calcification, par exemple avec du PBS environ 0,01 à 0,02 M, soit rincé à fond ou traité d'autre façon avec une solution hypophosphatée avant la greffe pour en éliminer le phosphate et ainsi réduire ou atténuer la calcification du tissu greffé selon l'invention. Le rinçage ou le traitement du tissu greffable contenant une quantité de phosphate entretenant la calcification avant la greffe, est de préférence réalisé avec une solution pratiquement dépourvue de phosphate.

Selon l'invention, la solution contient des cations divalents et est maintenue en contact avec lesdits ions, de sorte que le tissu présente une réduction ou une atténuation avantageuse de la calcification après la greffe.

Les ions divalents ajoutés au tissu semblent entrer en compétition de façon efficace vis-à-vis des sites de fixation du calcium du tissu, en particulier après la greffe, lorsque dans certains cas, des sites additionnels de fixation du calcium sont formés. L'accroissement de la quantité des ions divalents tels les ions magnésium dans le tissu après fixation dans du glutaraldéhyde-PBS, provoque une réduction ou une atténuation prolongée de la calcification, tandis qu'un tissu fixé dans du glutaraldéhyde-PBS avec des quantités accrues de cation divalent puis exposé à des quantités additionnelles de phosphate entretenant la calcification, ne présente pas de diminution ou d'atténuation de la calcification. Donc, selon l'invention, il n'est pas nécessaire de maintenir le tissu traité par le cation divalent en contact avec une solution hypophosphatée avant ou pendant le traitement par l'ion, cependant on préfère maintenir le tissu dans une solution hypophosphatée après le traitement par l'ion divalent avant la greffe.

Selon la présente invention, il est apparu que tout ion divalent qui entre en compétition de façon efficace vis-à-vis des sites de fixation du calcium dans le tissu réduit la calcification. Par conséquent, les cations divalents employés de préférence sont les ions Ba, Mg, Sr, Cu, Zn, Ag et Hg. On a constaté, conformément à l'invention, qu'un tissu traité par des ions Ba, Mg et Sr préalablement à l'implantation réduisent de façon efficace la calcification. L'ion Mg est employé de préférence dans le cadre de l'invention.

Dans un mode de réalisation préféré de l'invention, les ions magnésium proviennent de solutions de sels de magnésium ; on préfère particulièrement des solutions de sel soluble dans l'eau tel que le chlorure de magnésium ($MgCl_2$), le sulfate de magnésium ($MgSO_4$) et le carbonate de magnésium ($MgCO_3$). Dans le mode de réalisation que l'on préfère particulièrement, le sel de magnésium contient des ions magnésium en une quantité efficace pour réduire ou atténuer la calcification du tissu après la greffe. Bien que la concentration des ions magnésium et le moment de leur mise en contact avec le tissu puissent varier, il est préférable que le tissu soit pratiquement saturé de solutions contenant des quantités efficaces de cet ion.

Selon l'invention, on considère que les quantités efficaces de cations divalents tels que les ions magnésium en contact avec le tissu sont des quantités supérieures aux quantités observées dans certains PBS, les solutions salées équilibrées et le plasma. Les solutions classiques de PBS ont généralement une teneur en ions magnésium de l'ordre de 0,001 % en poids, les solutions salées équilibrées ont une teneur de l'ordre de 0,002 % en poids et la limite supérieure dans le plasma est de l'ordre d'environ 0,003 % en poids. La quantité préférée d'ions magnésium est supérieure à environ 0,003 à environ 0,004 % en poids. La quantité maximale d'ions magnésium considérée comme utile dans l'invention est celle nécessaire à l'obtention d'une solution isotonique. La saturation du tissu avec des solutions contenant environ 0,03 % d'ions magnésium réduit de façon efficace la calcification du tissu et constitue un procédé préféré. Pour effectuer cette saturation, on plonge le tissu dans une solution de chlorure de magnésium à 0,26 % en poids qui constitue une solution à 0,03 % en poids d'ions magnésium. Des solutions consistant en des solutions salines équilibrées contenant plus d'environ 0,003 % d'ions magnésium normalement présents dans le plasma conduisent à un effet bénéfique et sont employées de préférence.

Selon un mode de réalisation de l'invention, un tissu valvulaire porcin prélevé, expédié dans une solution salée tamponnée à l'HEPES, fixé dans une solution salée tamponnée à l'HEPES contenant 0,25 % en poids de chlorure de magnésium et 0,625 % en poids de glutaraldéhyde, rincé dans une solution salée tamponnée à l'HEPES contenant 0,26 % en poids de chlorure de magnésium, stérilisé dans une solution salée tamponnée à l'HEPES contenant 0,26 % en poids de chlorure de magnésium et environ 4,0 % en poids de formaldéhyde, rincé et conservé dans une solution salée tamponnée à l'HEPES contenant 0,26 % en poids de chlorure de magnésium et 0,625 % en poids de glutaraldéhyde, jusqu'à un moment précédant immédiatement la greffe, présente de façon avantageuse une atténuation ou une réduction importante de la calcification après la greffe. Donc selon un mode de réalisation de l'invention, on préfère particulièrement fixer le tissu biologique par le glutaraldéhyde dans une solution salée tamponnée à l'HEPES contenant une quantité efficace de magnésium et maintenir la solution en contact avec ladite solution salée tamponnée à l'HEPES et les ions magnésium jusqu'à un moment précédant immédiatement la greffe.

Egalement selon l'invention, le tissu biologique traité en l'absence de quantités efficaces de cations divalents tels que Mg peut être rincé dans des solutions stériles, telles que des solutions de chlorure de magnésium, immédiatement avant la greffe pour réduire la calcification ultérieure.

L'invention est illustrée par les exemples non limitatifs suivants.

Exemple I

On rince à fond un tissu de valvule aortique porcine prélevé, on l'expédie, on le fixe avec du glutaraldéhyde 0,625 % en poids, on le stérilise dans du formaldéhyde à environ 4 %, on le conserve entre environ 4 et 25 °C, le tout en présence d'une solution isotonique (285 ± 15 milliosmoles) hypophosphatée contenant 0,885 % en poids de chlorure de sodium à pH 7,3, on rince dans une solution salée pour éliminer le glutaraldéhyde résiduel immédiatement avant l'implantation et on implante à des lapins en cours de croissance. On récupère le tissu valvulaire à des intervalles réguliers d'une semaine pendant une durée totale de 6 semaines. Après récupération, on évalue l'importance de la calcification tissulaire par détermination quantitative du pourcentage pondéral de calcium dans le tissu sec par analyse par absorption atomique, et on l'évalue du point de vue histologique par observation du degré de calcification de coupes de tissu colorées selon la méthode de Von Kossa. De façon simultanée et identique, on évalue l'importance de la calcification d'un tissu de valvule cardiaque que l'on a rincé, expédié, fixé avec du glutaraldéhyde à 0,625 % en poids, stérilisé dans du formaldéhyde à environ 4 %, conservé entre environ 4 et 25 °C, le tout en présence d'une solution isotonique 0,02 M en phosphate et à 0,885 % en poids de chlorure de sodium à pH 7,3 (PBS 0,02 M), rincé dans une solution salée pour éliminer le glutaraldéhyde résiduel immédiatement avant l'implantation et implanté à des lapins en cours de croissance.

Les résultats histologiques et quantitatifs indiquent que le tissu valvulaire implanté traité avec la solution hypophosphatée présente une diminution nette de la calcification par rapport au tissu valvulaire traité avec du PBS 0,02 M.

Exemple II

On effectue des expériences identiques à celles de l'exemple I, si ce n'est que l'on implante le tissu à des lapins adultes et qu'on le récupère à des intervalles réguliers d'un mois pendant une durée totale de six mois. De façon semblable, les résultats de l'examen histologique et du dosage quantitatif montrent que le tissu valvulaire implanté traité avec la solution hypophosphatée présente une diminution importante de la calcification par rapport au tissu valvulaire traité avec du PBS 0,02 M.

Exemple III

On effectue des expériences semblables à celles de l'exemple II, si ce n'est que la solution hypophosphatée contient de plus 0,54 g/l du sel de sodium de l'HEPES lors du rinçage et de l'expédition et 5,83 g/l du sel de sodium de l'HEPES pendant la fixation, la conservation et la stérilisation. Dans ce cas également, les résultats de l'examen histologique et du dosage quantitatif indiquent que le tissu valvulaire implanté traité avec la solution hypophosphatée présente une calcification réduite par rapport au tissu valvulaire traité avec le PBS 0,02 M.

Exemple IV

On effectue des expériences semblables à celles de l'exemple I, si ce n'est que les solutions hypophosphatées contiennent de plus 0,54 g/l du sel de sodium de l'HEPES pendant le rinçage et l'expédition, 5,39 g/l du sel de sodium de l'HEPES pendant la fixation, le stockage et la stérilisation et 2,6 g/l de $MgCl_2$, $6H_2O$ pendant la fixation, la conservation et la stérilisation. Dans ce cas également, les résultats de l'examen histologique et des dosages quantitatifs montrent que le tissu valvulaire implanté traité avec la solution hypophosphatée présente une diminution nette de la calcification par rapport au tissu valvulaire traité avec le PBS 0,02 M.

Exemple V

On effectue des expériences identiques à celles de l'exemple IV, si ce n'est que l'on implante le tissu à des lapins adultes et qu'on le récupère à des intervalles réguliers d'un mois pendant une durée totale de six mois. Les résultats de l'examen histologique et du dosage quantitatif montrent que les tissus valvulaires implantés traités avec la solution hypophosphatée, présentent une diminution nette et prolongée de la calcification par rapport aux tissus valvulaires traités avec du PBS 0,02 M.

Exemple VI

On effectue des expériences semblables à celles de l'exemple I, si ce n'est que la solution hypophosphatée contient de plus du tampon cacodylate à la concentration de 0,5 M et que l'on récupère le tissu valvulaire uniquement après une et deux semaines. De plus, on évalue simultanément et de façon identique l'importance de la calcification observée lorsqu'avant la greffe, on utilise une solution 0,012 M en phosphate et à 0,885 % en poids de chlorure de sodium à pH 7,5 (PBS 0,012 M). Les résultats histologiques montrent que le tissu valvulaire implanté traité avec une solution hypophosphatée présente une légère diminution de la calcification après une semaine par rapport au tissu valvulaire traité avec le PBS 0,012 M. Ensuite, on observe une calcification modérée.

Exemple VII

On effectue des expériences identiques à celles de l'exemple VI, si ce n'est que l'on remplace le cacodylate par du tampon borate 0,1 M. Les résultats histologiques sont semblables à ceux de l'exemple 6.

Exemple VIII

On effectue des expériences identiques à celles de l'exemple VII, si ce n'est qu'aucune des solutions comparatives n'est hypophosphatée, les solutions utilisées pour la fixation, la conservation et la stérilisation étant constituées de PBS 0,02 % au lieu de tampon HEPES. Les résultats de l'examen histologique et du dosage quantitatif montrent que le tissu valvulaire implanté traité avec la solution de chlorure de magnésium phosphatée présente une réduction de la calcification par rapport au tissu valvulaire traité sans chlorure de magnésium.

Exemple IX

On effectue des expériences semblables à celles de l'exemple III, pour évaluer l'intégrité du tissu après l'implantation. Les résultats des analyses indiquent qu'il n'y a pas de différence significative de la température de rétraction, des valeurs de la réaction à la ninhydrine, de la teneur en acide uronique lixiviable dans le produit de lixiviation acide des mycopolysaccharides, de la stabilité du tissu soumis à une digestion par la pronase, de la coloration histologique, de l'analyse des aminoacides, de l'ultrastructure évaluée par microscopie électronique par transmission et de la teneur en humidité.

Exemples X à XXII

On traite du tissu valvulaire cardiaque porcin prélevé, avec des solutions hypophosphatées ou des solutions contenant du phosphate à divers stades du traitement (avant fixation, pendant la fixation avec le glutaraldéhyde et après la fixation), avant l'implantation à des rats pour évaluer le degré d'atténuation de la calcification obtenu pour chaque stade du traitement. Les résultats du tableau suivant résument les observations faites pendant une période atteignant dans certains cas deux mois après l'implantation. Dans le tableau, $G_p$ indique le tissu traité par le glutaraldéhyde dans du PBS 0,02 M ; $F_{H20}$ indique le tissu traité par du formaldéhyde aqueux ; $G_{eau}$ indique le tissu traité par le glutaraldéhyde aqueux ; $F_p$ indique le tissu traité par le formaldéhyde dans du PBS 0,02 M ; $G_{HEPES}$ indique le tissu traité par le glutaraldéhyde dans de l'HEPES 0,002 M ; $G_{carbonate}$ indique le tissu traité par le glutaraldéhyde dans du tampon carbonate ; solution salée/HEPES désigne la solution salée tamponnée par l'HEPES à 0,002 M et BSS indique une solution salée équilibrée.

Tableau

Etude de la calcification chez le rat

| Avant fixation | Traitement de fixation | Après fixation | Degré de cal- cification |
|---|---|---|---|
| PBS | $G_p$ | phosphatée | important |
| PBS | $F_{eau}$ | hypophosphatée | réduit (dégénérescence) |
| PBS | $F_p$ | phosphatée | important (dégénérescence) |
| PBS | $G_{eau}$ | hypophosphatée | réduit |
| PBS | $G_{HEPES}$ | hypophosphatée | réduit |

8

(Fortsetzung)

| Avant fixation | Traitement de fixation | Après fixation | Degré de calcification |
|---|---|---|---|
| PBS | $G_{carbonate}$ | hypophosphatée | légèrement réduit |
| HANKS (BSS) | $G_p$ | phosphatée | important |
| Solution salée HEPES | $G_p$ | phosphatée | important |
| HANKS (BSS) | G solution salée/HEPES | hypophosphatée | réduit |
| PBS Solution salée | G solution salée/HEPES | hypophosphatée | réduit |
| Solution salée | G solution salée/HEPES | hypophosphatée | réduit (prolongé) |
| PBS | $G_p$ | $MgCl_2$ hypophosphatée | réduit (prolongé) |
| PBS | $G_p$ | $MgCl_2$-$G_p$ | important |

## Exemple XXIII

Des essais identiques à ceux de l'exemple IV sont mis en œuvre, si ce n'est que l'on remplace $MgCl_2$, $6H_2O$ par $BaCl_2$, $2H_2O$ à une concentration de 2,2 g/l. Les résultats obtenus montrent que Ba a un effet sur la calcification similaire à celui du Mg.

## Exemple XXIV

Des essais identiques à ceux de l'exemple IV sont mis en œuvre, si ce n'est que l'on remplace $MgCl_2$, $6H_2O$ par $SrCl_2$, $2H_2O$ à une concentration de 2,3 g/l. Les résultats obtenus montrent que Sr a un effet sur la calcification similaire à celui du Mg.

## Revendications

1. Procédé pour le traitement et la conservation d'un tissu biologique destiné à être greffé sur un homme ou un animal et la diminution ou atténuation prolongée de la calcification après la greffe, caractérisé en ce qu'il consiste à mettre en contact ledit tissu avec une solution isotonique hypophosphatée efficace pour réduire la calcification du tissu, ladite solution isotonique comprenant au moins un sel ou tampon physiologiquement acceptable ayant un pH compris entre 7,0 et 7,6 et plus de 0,003 % en poids d'un cation divalent autre que le calcium entrant en compétition vis-à-vis des sites de fixation du calcium, et à conserver ledit tissu dans ladite solution jusqu'à son utilisation.

2. Procédé selon la revendication 1, caractérisé en ce que le cation divalent est un ion magnésium ou un de ses sels.

3. Procédé selon la revendication 2, caractérisé en ce que le sel de magnésium est $MgCl_2$, $MgSO_4$ et $MgCO_3$.

4. Procédé selon la revendication 3, caractérisé en ce que le sel de magnésium est le chlorure de magnésium.

5. Procédé selon la revendication 1, caractérisé en ce que ladite solution est une solution salée, éventuellement tamponnée, contenant de 0,2 à 6,0 % en poids de glutaraldéhyde et de préférence 0,5 à 0,7 % en poids de glutaraldéhyde.

6. Procédé selon une quelconque des revendications 1 à 5, caractérisé en ce que ladite solution est constituée de solution salée tamponnée 0,02 M en HEPES et contenant environ 0,625 % en poids de glutaraldéhyde à un pH d'environ 7,1 à environ 7,5.

9

7. Procédé selon l'une quelconque des revendications 1 à 6, caractérisé en ce que la quantité du cation divalent autre que le calcium présente est suffisante pour en saturer le tissu.

8. Procédé selon l'une quelconque des revendications 1 à 7, caractérisé en ce que le tissu biologique est destiné à être employé pour la préparation d'une valvule cardiaque.

9. Procédé selon l'une quelconque des revendications 1 à 8, caractérisé en ce que la concentration du chlorure de magnésium dans ladite solution est d'environ 0,26 % en poids.

10. Bioprothèse stable obtenue à l'aide du procédé selon l'une quelconque des revendications 1 à 9.

11. Valvule cardiaque obtenue à l'aide du procédé selon l'une quelconque des revendications 1 à 9.

## Claims

1. Process for treating and preserving a biological tissue intended to be grafted upon a human or animal subject and the reduction or extended attenuation of calcification after the grafting operation, characterized in that it consists in putting into contact the said tissue with an effective hypophosphated isotonic solution in order to reduce calcification of the tissue, the said isotonic solution comprising at least one physiologically acceptable salt or buffer having a pH comprised between 7.0 and 7.6 and more than 0.003 % by weight of a divalent cation other than calcium entering into competition with respect to the fixation sites of the calcium, and in preserving the said tissue in the said solution until its utilization.

2. Process according to claim 1, characterized in that the divalent cation is a magnesium ion or one of its salts.

3. Process according to claim 2, characterized in that the magnesium salt is $MgCl_2$, $MgSO_4$ and $MgCO_3$.

4. Process according to claim 3, characterized in that the magnesium salt is magnesium chloride.

5. Process according to claim 1, characterized in that the said solution is a salt solution, possibly buffered, containing from 0.2 to 6.0 % by weight of glutaraldehyde and preferably from 0.5 to 0.7 % by weight of glutaraldehyde.

6. Process according to any one of claims 1 to 5, characterized in that the said solution is constituted by a salt solution buffered at 0.02 M in HEPES and containing about 0.625 % by weight of glutaraldehyde at a pH of about 7.1 to about 7.5.

7. Process according to any one of claims 1 to 6, characterized in that the existing quantity of divalent cation other than the calcium is sufficient to saturate the tissue.

8. Process according to any one of claims 1 to 7, characterized in that the biological tissue is intended to be used for preparing a cardiac valvula.

9. Process according to any one of claims 1 to 8, characterized in that the concentration of magnesium chloride in the said solution is about 0.26 % by weight.

10. Stable bioprosthesis obtained by utilizing the process according to any one of claims 1 to 9.

11. Cardiac valvula obtained by utilizing the process according to any one of claims 1 to 9.

## Patentansprüche

1. Verfahren zur Behandlung und Aufbewahrung eines auf einen menschlichen oder tierischen Organismus aufzupflanzenden biologischen Gewebes und zur Verminderung oder andauernden Abschwächung der Verkalkung nach der Verpflanzung, dadurch gekennzeichnet, dass man das Gewebe mit einer die Gewebeverkalkung vermindernden isotonischen Hypophosphatlösung in Berührung bringt, die wenigstens ein physiologisch ertragbares Puffersalz mit einem pH-Wert von 7,0 bis 7,6 und mit mehr als 0,003 Gew.-% eines zweiwertigen Kations ausschliesslich Kalzium, welches hinsichtlich der Kalziumfixierstellen wirksam ist, und dass man das Gewebe bis zum Zeitpunkt seiner Verwendung in dieser Lösung aufbewahrt.

2. Verfahren nach Anspruch 1, dadurch gekennzeichnet, dass das zweiwertige Kation ein Magnesiumion oder Magnesiumsalz ist.

3. Verfahren nach Anspruch 2, dadurch gekennzeichnet, dass das Magnesiumsalz $MgCl_2$, $MgSO_4$ und $MgCO_3$ ist.

4. Verfahren nach Anspruch 3, dadurch gekennzeichnet, dass das Magnesiumsalz Magnesiumchlorid ist.

5. Verfahren nach Anspruch 1, dadurch gekennzeichnet, dass das die Lösung eine gegebenenfalls gepufferte salzige Lösung ist, die 0.2 bis 6,0 Gew.-% Glutaraldehyd, vorzugsweise 0,5 bis 0,7 Gew.-% Glutaraldehyd enthält.

6. Verfahren nach einem der Ansprüche 1 bis 5, dadurch gekennzeichnet, dass die Lösung eine auf 0,02 M gepufferte HEPES-Lösung ist, die 0,625 Gew.-% Glutaraldehyd enthält, mit einem pH-Wert von etwa 7,1 bis etwa 7,5.

7. Verfahren nach einem der Ansprüche 1 bis 6, dadurch gekennzeichnet, dass die vorliegende Menge des von Kalzium verschiedenen zweiwertigen Kations zur Sättigung des Gewebes hinreichend ist.

8. Verfahren nach einem der Ansprüche 1 bis 7, dadurch gekennzeichnet, dass das biologische Gewebe zur Anfertigung einer Herzklappe bestimmt ist.

9. Verfahren nach einem der Ansprüche 1 bis 8, dadurch gekennzeichnet, dass die Magnesiumchloridkonzentration in der Lösung etwa 0,26 Gew.-% beträgt.

10. Vermittels des Verfahrens nach einem der Ansprüche 1 bis 9 hergestellte Bioprothese.

11. Vermittels des Verfahrens nach einem der Ansprüche 1 bis 9 hergestellte Herzklappe.